# FASCICULE DE BREVET EUROPEEN

(11) **EP 2 203 477 B1**
(45) Date de publication et mention de la délivrance du brevet: **28.05.2014**
(21) Numéro de dépôt: 08843245.5
(22) Date de dépôt: 07.10.2008
(51) Int. Cl.: G01N 33/569, A61K 31/737, C07K 16/10

(54) **ANTICORPS MONOCLONAUX SPECIFIQUES DE L'HEMAGGLUTININE DU VIRUS GRIPPAL**
FÜR GRIPPEVIRUS HÄMAGGLUTININ SPEZIFISCHE MONOKLONALE ANTIKÖRPER
INFLUENZA VIRUS HAEMAGGLUTININ-SPECIFIC MONOCLONAL ANTIBODIES

(30) Priorité: 08.10.2007 FR 0758134
(43) Date de publication de la demande: 07.07.2010
(73) Titulaire: Sanofi Pasteur, 69367 Lyon Cedex 07 (FR)
(72) Inventeur: MOSTE, Catherine, F-69260 Charbonnières les Bains (FR); LEGASTELOIS, Isabelle, F-69700 Saint Andéol le Château (FR); CHEVALIER, Michel, F-38270 Beaurepaire (FR); THION, Laurent, F-69240 Bourg de Thizy (FR)
(86) Numéro de dépôt international: PCT/FR2008/051810
(87) Numéro de publication internationale: WO 2009/053604

(56) Documents cités:
- WO-A1-2005/118645
- WO-A1-2006/101000
- US-A- 4 625 015
- BIANCHI ELISABETTA ET AL: "Universal influenza B vaccine based on the maturational cleavage site of the hemagglutinin precursor" JOURNAL OF VIROLOGY, THE AMERICAN SOCIETY FOR MICROBIOLOGY, US, vol. 79, no. 12, 1 juin 2005 (2005-06-01), pages 7380-7388, XP002445847 ISSN: 0022-538X
- GALILI U ET AL: "Enhancement of antigen presentation of influenza virus hemagglutinin by the natural human anti-Gal antibody" 1 mars 1996 (1996-03-01), VACCINE, BUTTERWORTH SCIENTIFIC. GUILDFORD, GB, PAGE(S) 321 - 328 , XP004057331 ISSN: 0264-410X le document en entier
- SCHWARZ R T ET AL: "Carbohydrates of influenza virus IV. Strain-dependent variations" 1 septembre 1981 (1981-09-01), VIROLOGY, ACADEMIC PRESS,ORLANDO, US, PAGE(S) 584 - 593 , XP023048592 ISSN: 0042-6822 [extrait le 1981-09-01] le document en entier

## Description

L'invention est relative à un anticorps monoclonal reconnaissant une structure antigénique glycosylée présente à la fois sur l'hémagglutinine des virus grippaux de type A et de type B, ainsi qu'à leur utilisation à des fins diagnostiques, thérapeutiques ou à des fins de purification.

Il existe 3 types de virus grippaux (types A, B et C) responsables de pathologies infectieuses chez l'homme et les animaux. Actuellement, les virus de types A et B sont les agents responsables des épidémies et des pandémies de grippe observées chez l'homme.

Les virus de type A circulant principalement chez l'homme et les oiseaux sont subdivisés en sous types en fonction de la structure antigénique de l'hémagglutinine (l'HA) et de la neuraminidase (NA) qui sont les principaux constituants de l'enveloppe virale. On distingue 16 sous types d'HA (H1 à H16) et 9 sous types de NA (N1 à N9). Le sous type d'un virus de type A est défini par le sous type d'HA et le sous type de NA qu'il porte. Les sous types de virus H1N1 et H3N2 sont ceux qui circulent actuellement chez l'homme. On craint que le sous type H5N1 ou le sous type H7N1 qui circulent chez les oiseaux s'adaptent à l'homme et soient à l'origine d'une nouvelle pandémie. On n'exclut pas non plus que le sous type H2N2 qui circulait entre les années 1957 et 1968 chez l'homme, réapparaisse et soit également à l'origine d'une pandémie chez les sujets de moins de 40 ans.

Les souches de virus de type B sont strictement humains. Les variations antigéniques de l'HA au sein des souches de type B sont plus faibles que celles observées au sein des souches de type A. Les souches de type B, isolées depuis les années 1970, sont divisées en deux groupes phylogénétiques et antigéniques distincts en fonction de la séquence génétique de l'HA (Lindstrom SE, Journal of Virology, 1999, pp 4413-4426). La souche B/Victoria/2/87 (B/victoria) est le chef de file du premier groupe tandis que la souche B/Yamagata/16/88 (B/Yagamata) est le chef de file du second groupe. Les souches de virus de type B qui ont été isolées dans les années 1980 appartiennent surtout au groupe B/Victoria tandis que les souches qui ont été isolées dans les années 1990 appartiennent surtout au groupe B/Yamagata. En 1994, le groupe B/Victoria a ré-émergé en Chine et depuis il arrive qu'on isole des souches de type B appartenant aux deux groupes durant une même saison grippale (Nakagawa N et al., Journal of Medical Virology, 2001, pp754-750).

L'HA du virus grippal est, dans sa forme native, une glycoprotéine trimérique d'un poids moléculaire d'environ 220000 daltons. Elle est soumise à une forte « pression de sélection » avec comme conséquence l'apparition :
- de variations majeures dans sa structure entrainant une cassure antigénique ou « antigenic shift »). On observe ces phénomènes dans les virus de type A et sont à l'origine de l'apparition de virus réassortis ayant un nouveau sous type d'HA. Ces virus réassortis peuvent être à l'origine de nouvelles pandémies chez l'homme ou les animaux ;
- de variations mineures dans sa structure dues principalement à des mutations ponctuelles entrainant un glissement antigénique ou « antigenic drift »). On observe ces phénomènes aussi bien dans les virus de type A que dans les virus de type B. Elles sont à l'origine de l'apparition de nouvelles souches virales qui peuvent déclencher des épidémies ou des foyers de grippe sporadiques pendant les périodes inter pandémiques.

L'HA du virus grippal contient également de nombreux sites de glycosylation (entre 7 et 9 sur les sous types H1 et H3). Les chaînes glycosylées sont reliées à la structure protéique par l'intermédiaire de liaisons N-glycosidiques qui relient un asparagine de la séquence protéique à un N-acétylglucosamine de la chaîne sucrée. On distingue classiquement 2 types de chaînes glycosylées en fonction de leur composition en sucres : les chaînes glycosylées de type I contiennent essentiellement du N acetyl-glucosamine, du mannose, du galactose et du fucose et les chaînes glycosylées de type II contiennent essentiellement du mannose et du N acetyl-glucosamine. La N glycosylation de l'HA se fait par l'intermédiaire du dolichol phosphate qui transfère en bloc la séquence oligo saccharidique suivante : (glucose)₃-(mannose)₉-(N-acetyl-glucosamine)₂ sur les asparagines qui se trouvent dans une séquence consensus de type Asn-X-Ser/Thr de la chaîne protéique (Virology, 133, 77-91 (1984)). La séquence oligosaccharidique transférée en bloc a une configuration tri-antennée représentée schématiquement de la façon suivante :

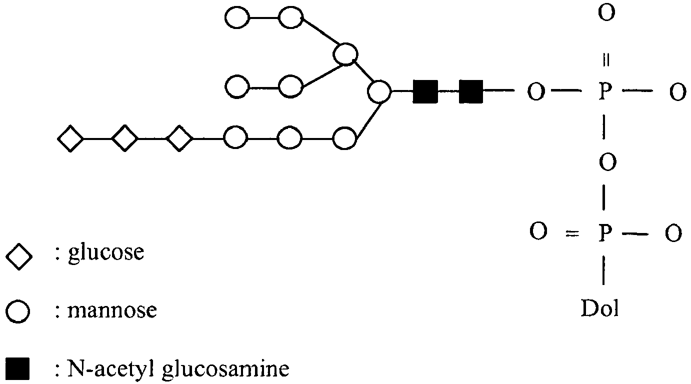
O
=
O — P — O
|
O
|
O = P — O
|
Dol
: glucose
: mannose
: N-acetyl glucosamine

Comme cela été montré par Mir-Shekari S.Y. et coll. (Journal of biological Chemistry, 272, 4027-4036 (1997), les séquences initiales tri-antennées transférées subissent ensuite individuellement un processus de transformation en fonction de leur site d'ancrage sur la chaîne protéique de l'HA. Les structures glycosylées finalement peuvent être bi-antennées, tri-antennées, ou tétra-antennées avec un nombre plus ou moins important de sucres sur chacune des antennes.

Pour prévenir les épidémies de grippe, on prépare annuellement des vaccins contenant deux souches de virus A appartenant à des sous types différents (actuellement les sous types H1N1 et H3N2) et une souche de virus de type B. Les souches vaccinales utilisées prennent en compte les variations qui se sont produites d'une année sur l'autre au niveau de la séquence protéique de l'HA et qui sont à l'origine de l'apparition de nouveaux foyers épidémiques. On identifie et on caractérise ces nouvelles souches à l'aide d'outils diagnostiques très précis. notamment au moyen d'anticorps monoclonaux qui mettent en évidence de façon très spécifique les variations observées. Ces anticorps monoclonaux ont une spécificité très étroite puisqu'ils reconnaissent généralement l'HA d'une seule souche de virus. Ils ont une spécificité dite « de souche ».

Il existe également des anticorps monoclonaux ayant une spécificité plus large dite de « sous type » pour caractériser les virus de type A. Ils sont spécifiques de « sous type » dans la mesure où ils reconnaissent les HA de souches de virus ayant le même sous type d'HA mais pas les HA de souches virales d'un autre sous type. Il existe notamment des anticorps monoclonaux spécifiques du sous type H1 ou du sous type H3. Il a été rapporté dans US 5,589,174 un anticorps monoclonal cross réactif reconnaissant à la fois les HA des virus grippaux appartenant aux sous types H1N1 et H2N2. US 4,625,015 décrit des immun sérums obtenus par immunisation avec des peptides synthétiques correspondant à certaines régions de l'HA du virus grippal X-47 qui reconnaissent à la fois des souches de virus de type A ayant le sous type H3N2 et le sous type H1N1 ainsi qu'une souche de virus de type B. Par contre les immuns sérums obtenus après immunisation avec le virus X-47 entier ont un spectre de reconnaissance plus restreint puisqu'ils reconnaissent uniquement des souches de virus de type A ayant le sous type H3N2 et le sous type H1N1.

En ce qui concerne les anticorps monoclonaux dirigés contre l'HA des virus de type B, Nakagawa N. a décrit une batterie d'anticorps monoclonaux dirigés contre l'HA des souches appartenant aux deux groupes B/Victoria et B/Yagamata. Les anticorps monoclonaux 10B8, 8E6, 1H12, 2H12 et 9E10 reconnaissent l'HA des souches de virus de type B appartenant au groupe B/Victoria qui ont été isolées entre 1996 et 1997 mais pas l'HA des souches de virus de type B isolées durant cette même période et qui appartiennent au groupe B/Yamagata. (Nakagawa N et al., Journal of Virological methods (1999), 113-120). Ces anticorps monoclonaux ont été obtenus en immunisant des souris avec la souche B/Nagasaki/1/87 qui appartient au groupe B/Victoria. Il a décrit également des anticorps monoclonaux 1B2, 5B1, 5H4, 7H11, 8B3 et 9G6 obtenus après immunisation de souris avec la souche B/Mie/1/93 (qui appartient au groupe B/Yagamata) reconnaissant l'HA de souches de virus de type B appartenant au groupe B/Yamagata (Nakagawa N et al., Journal of Medical Virology (2001), 745-750). A notre connaissance, on n'a jamais décrit d'anticorps monoclonaux reconnaissant à la fois l'HA des souches appartenant au groupe B/Victoria et l'HA des souches appartenant au groupe B/Yagamata.

Même si l'utilisation d'anticorps monoclonaux ayant une spécificité restreinte s'avère très précieuse pour suivre les variations antigéniques de l'HA qui se produisent régulièrement au sein des souches virales circulantes, il peut s'avérer également très utile de disposer d'anticorps monoclonaux qui ont un large spectre de reconnaissance des virus grippaux. On détecte ainsi facilement et rapidement la présence d'un virus grippal dans un matériel biologique sans qu'il soit nécessaire d'utiliser toute une batterie d'anticorps pour le mettre en évidence. Ils peuvent également être utilisés comme alternative aux anticorps monoclonaux spécifiques de souches qui sont habituellement utilisés pour contrôler les vaccins anti grippaux lorsque ceux-ci ne sont pas disponibles dans des délais suffisamment courts.

La présente invention répond à ce besoin et a pour objet un anticorps monoclonal spécifique de l'hémagglutinine du virus grippal, reconnaissant une structure antigénique présente à la fois sur les sous types H1 et H3 des hémagglutinines des virus grippaux de type A et sur l'hémagglutinine des virus grippaux de type B.

L'anticorps monoclonal selon l'invention est bien spécifique de l'HA du virus grippal car il ne reconnait pas les autres composants du virus grippal, notamment la NA, la protéine M ou la protéine NP. De plus, il inhibe au moins l'activité hémagglutinante médiée par des souches de virus grippal de type A appartenant à un même sous type. Son spectre de reconnaissance vis-à-vis des hémagglutinines du virus grippal est par contre très large. Il est plus large que les anticorps monoclonaux qui ont une spécificité dite de « sous type » (spécificité limitée à un sous type d'HA des virus de type A) ou de « type » (spécificité limitée aux HA d'un seul type de virus). L'anticorps monoclonal selon l'invention reconnait en effet à la fois les hémagglutinines des virus de type A et B.

Vis-à-vis des virus de type A, l'anticorps monoclonal selon l'invention reconnait un déterminant antigénique présent sur les sous types H1 et H3. Il reconnait notamment des souches de virus appartenant au sous type H1N1, comme par exemple la souche A/New Caledonia/20/99, et/ou la souche A/Chile/1/83X83 et des souches de virus appartenant au sous type H3N2, comme par exemple la souche A/Panama/2007/99, la souche A/Wisconsin/67/2005, la souche A/new York/55/2004, la souche A/Wyoming/03/2003, la souche A/Honk Kong/1/68, la souche A/Beijing/32/92, la souche A/Shangdong/9/93, la souche A/Johannesburg/33/94, la souche A/Nanchang/933/95, et/ou la souche A/Sydney/5/97.

Vis-à-vis des virus grippaux de type B, il reconnait aussi une structure antigénique présente sur l'HA des souches de virus de type B. Il reconnait notamment la souche B/Shangdong/7/97, la souche B/Jiangsu/10/03, la souche B/Brisbane 23/02, la souche B/Yagamata 16/88, la souche B/Beijing 1/87, et/ou la souche B/Malaysia/2506/04. Il reconnait généralement à la fois des souches de virus de type B appartenant au groupe B/victoria (comme la souche B/Shangdong/7/97) et des souches de virus de type B appartenant au groupe B/yagamata (comme la souche B/Jiangsu/10/03).

L'invention a donc également pour objet un anticorps monoclonal spécifique de l'HA du virus grippal, reconnaissant un oligosaccharide contenant un galactose sulfate présente à la fois sur la partie N-glycosylée des sous types H1 et H3 des hémagglutinines des virus grippaux de type A et de l'hémagglutinine des virus grippaux de type B, les dits virus avant été produits sur du matériel biologique d'origine aviaire. Plus particulièrement, l'anticorps monoclonal selon l'invention reconnait l'hémagglutinine des virus grippaux de type B-appartenant au groupe B/victoria et/ou au groupe B/yagamata.

Un anticorps monoclonal selon l'invention reconnait donc spécifiquement l'HA d'au moins une souche de virus grippal de type A ayant le sous type H1N1, l'HA d'au moins une souche de virus grippal de type A ayant le sous type H3N2, l'HA d'au moins une souche de virus grippal de type B appartenant au groupe B/Victoria et l'HA d'au moins une souche de virus grippal de type B appartenant au groupe B/Yagamata.

Généralement, un anticorps monoclonal selon l'invention reconnait les HA de plusieurs souches de virus grippal de type A ayant le sous type H1N1, les HA de plusieurs souches de virus grippal de type A ayant le sous type H3N2, les HA de plusieurs souches de virus grippal de type B appartenant au groupe B/Victoria et les HA de plusieurs souches de virus grippal de type B appartenant au groupe B/Yagamata. Comme souches de virus de type H1N1 qui sont reconnues par un anticorps monoclonal selon l'invention, on indique les souches A/New Caledonia/20/99 et A/Chile/1/83/X83. Comme souches de virus de types H3N2 qui sont reconnues par un anticorps monoclonal selon l'invention, on indique les souches A/wyoming/3/03 et A/NewYork/55/04. Comme souches de virus de type B appartenant au groupe B/victoria qui sont reconnues par un anticorps monoclonal selon l'invention, on indique les souches B/Shandong/7/97 et B/Brisbane /32/02. Comme souches de virus de type B appartenant au groupe B/Yagamata qui sont reconnues par un anticorps monoclonal selon l'invention, on indique les souches B/Shandong/7/97 et B/Brisbane /32/02. Comme souches de virus de type B appartenant au groupe B/Yagamata qui sont reconnues par un anticorps monoclonal selon l'invention, on indique les souches B/Yagamata/16/88 et B/Jiangsu/10/03. On indique que les anticorps monoclonaux Y6F5 et Y13F9 présentent ces caractéristiques de reconnaissance (Cf. exemple 1.2).

De façon préférée, un anticorps monoclonal selon l'invention reconnait une structure antigénique qui est présente à la fois, sur les HA de plus de deux souches de virus grippal de type A ayant le sous type H1N1, sur les HA de plus de deux souches de virus grippal de type A ayant le sous type H3N2, sur les HA de plus de deux souches de virus grippal de type B appartenant au groupe B/Victoria et sur les HA de plus de deux souches de virus grippal de type B appartenant au groupe B/Yagamata.

De façon particulière, un anticorps monoclonal selon l'invention reconnait des souches virales dont les HA appartiennent au sous type H5 et/ou H7 qui pourraient être impliquées dans le développement de souches grippales pandémiques. L'anticorps monoclonal reconnait notamment la souche A/Vietnam/1194/04 NIBRG14 qui est une souche aviaire de sous type H5N1, accessible auprès du laboratoire National Institute for Biological Standards and Controls (NIBSC) et qui a été obtenue par génétique inverse comme cela est décrit par Nicolson et coll. dans Vaccine (2005), 23 :2943-2952. Les souches de virus ayant le sous type H5 ou H7 infectent surtout les populations aviaires mais on craint que de nouvelles mutations se produisent au niveau de la séquence protéique de l'HA et que ces souches mutantes deviennent infectieuses pour l'homme et être à l'origine du développement d'une pandémie grippale. Il y a donc un grand intérêt à pouvoir détecter et/ou à doser les HA ayant le sous type H5 ou H7.

Les souches virales de type A et B reconnues par un anticorps monoclonal selon l'invention sont produites en utilisant du matériel biologique d'origine aviaire. Le matériel biologique d'origine aviaire comprend les cellules, les tissus, les organes. les exsudats ou des extraits de ceux ci dans lesquels le virus grippal s'est multiplié. Habituellement, le matériel biologique d'origine aviaire provient de la poule ou du poulet. Il s'agit notamment de cultures de fibroblastes embryonnaires de poulets ou de cellules primaires d'embryons de poulets.

De façon particulière, le matériel biologique d'origine aviaire contenant le virus grippal est le liquide allantoïque de l'oeuf de poule lorsque le virus est produit sur des oeufs embryonnés. Ce matériel est utilisé notamment par fabriquer des vaccins contre la grippe. Selon les cas on utilise du matériel brut ou du matériel biologique purifié renfermant du virus purifié ou des composants purifiés de celui-ci.

Par conséquent les souches de virus grippaux dont les hémagglutinines sont reconnues par un anticorps monoclonal selon l'invention sont produits sur du matériel biologique d'origine aviaire.

On utilise habituellement les techniques immuno-enzymatiques bien connues de l'homme du métier et simples à mettre en oeuvre, telles que l'ELISA direct ou indirect, les techniques de dot-blotting, de western blotting ou de résonnance plasmonique de surface pour montrer que l'anticorps monoclonal selon l'invention reconnait les différents sous types d'HA (H1, H3, H5 et/ou H7 pour les virus grippaux de type A ainsi que les HA provenant des groupes B/Victoria et B/Yagamata pour les virus grippaux de type B).

La structure antigénique reconnue par un anticorps monoclonal selon l'invention a été localisée. L'antigène se situe sur la partie N-glycosylée des différentes HA reconnues par l'anticorps monoclonal: Un traitement de l'HA du virus grippal par la N-asparaginase (PNGase) entraine une perte de la reconnaissance.

L'invention a donc également pour objet :
Un anticorps monoclonal reconnaissant une structure antigénique, selon laquelle la structure antigénique est un enchaînement de sucres qui se trouve sur la partie N-glycosylée de l'hémagglutinine.
Ceci résulte du fait que la reconnaissance de la structure antigénique par l'anticorps monoclonal disparait lorsque l'hémagglutinine du virus de la grippe est traitée par la N-asparaginase, qui coupe spécifiquement les liaisons N-glycosidiques.

La «partie N-glycosylée de l'hémagglutinine» est définie comme étant toute chaîne de sucres reliée à la séquence d'acides aminés de l'hémagglutinine du virus de la grippe au moyen d'une liaison N-glycosidique qui lie un asparagine de cette séquence au N-acetyl glucosamine de la chaîne de sucres.

L'enchainement de sucres reconnu par un anticorps monoclonal selon l'invention est un oligosaccharide contenant un petit nombre de sucres qui est généralement compris entre 3 et 30 sucres, de façon préférée entre 3 et 20 sucres et de façon encore plus préférée entre 3 et 10 sucres.

Le terme « d'environ » qui est employé dans le texte est utilisé ici par référence à une valeur mesurable, telle qu'une quantité, un poids, une durée dont les valeurs peuvent varier en fonction de la précision de la technique de mesure utilisée. Ces valeurs peuvent varier de ± 20% pour les techniques de mesure les moins précises à ± 1% pour les techniques de mesure les plus précises. Le terme «d'environ» recouvre par conséquent des variations de ± 20%, de ± 10%, de façon préférée des variations de ± 5%, de façon plus préférée de ± 1% par rapport à la valeur cible mesurée.

Au moyen d'un anticorps monoclonal selon l'invention, dénommé Y6F5, on a isolé et caractérisé la structure antigénique glycosylée reconnue en mettant en oeuvre le procédé suivant : le virus produit sur oeufs embryonnés a été purifié selon des méthodes classiques de l'homme de métier. On a ensuite extrait l'HA en traitant le virus purifié à la bromélaïne. Puis on a dénaturé l'HA de façon à ce que les structures glycosylées soient plus sensibles à l'action de la PNGase. A cet effet, on a linéarisé la structure protéique par un traitement à l'aide d'un détergent tel que le dodecyl sulfate de sodium (SDS) ; puis on a cassé les ponts di sulfures par un traitement à l'aide d'un agent réducteur tel que le dithiotréhitol (DTT) suivi d'une alkylation au moyen de l'iodoacétamide. L'HA dénaturée a ensuite été traitée à la PNGase : les protéines ont été précipitées tandis que le surnageant qui contient le mélange de glycanes a été analysé en utilisant l'anticorps monoclonal Y6F5 préalablement fixé sur le « sensorship » d'un Biacore 3000. L'anticorps monoclonal retient uniquement les structures glycosylées qu'il reconnait. Grâce à l'appareillage employé, on a ensuite pu désorber au moyen de la soude la structure antigénique qui a été retenue par l'anticorps. La structure antigénique a ensuite été analysée par spectrométrie de masse. Les analyses par spectrométrie de masse MALDI-TOF ont montré qu'il s'agissait d'un oligosaccharide possédant un groupement sulfate ayant les caractéristiques suivantes: Le groupement sulfate est fixé à un galactose ; son poids moléculaire déterminé par spectrométrie de masse MALDI-TOF est d'environ 1240 daltons aux erreurs près d'incertitude des mesures de l'appareil (environ +/- 10 daltons).

L'anticorps monoclonal Y6F5 qui a servi à l'identification de cette structure chimique est produit par l'hybridome Y6F5 qui a été déposé le 10 Juillet 2007 à la Collection Nationale De Cultures de Microorganismes de l'INSTITUT PASTEUR de Paris (France) sous le référence d'identification Y6F5 et sous le numéro d'enregistrement CNCM I-3787.

Les anticorps monoclonaux selon l'invention sont produits habituellement à partir d'hybridomes que l'on obtient en fusionnant des splénocytes de souris hyperimmunisées avec une solution d'HA purifié provenant d'une souche de virus grippal de type A avec des cellules de myélome murin (Sp2/O-Ag14, p3x63Ag8, p3x63-Ag8.653,...) selon des méthodes bien connues de l'homme du métier. Les anticorps monoclonaux selon l'invention sont généralement des anticorps monoclonaux murins. Ces anticorps monoclonaux sont généralement testés par ELISA pour évaluer leur capacité à reconnaître les HA de souches appartenant au sous type H3N2 mais également leur capacité à reconnaitre les HA de souches appartenant au sous type H1N1 ainsi que les HA de souches de virus de type B. On sélectionne ceux qui reconnaissent à la fois des souches de virus de type A appartenant au sous type H1N1, des souches de virus de type A appartenant au sous type H3N2 et des souches de virus de type B.

On vérifie également qu'ils inhibent le pouvoir hémagglutinant médié par des souches virales appartenant au même sous type que la souche virale qui a servi à l'extraction de l'HA utilisée pour l'immunisation des souris. Par exemple, si les anticorps monoclonaux sont obtenus à partir de souris immunisées avec une solution d' HA provenant d'une souche ayant le sous type H3N2, on vérifie qu'ils inhibent le pouvoir hémagglutinant des souches H3N2.

On s'assure également qu'ils ne reconnaissent pas les autres protéines du virus grippal, en particulier la neuraminidase (NA), la protéine M et la protéine NP.

On complète la caractérisation de ces anticorps monoclonaux en étudiant leur comportement vis-à-vis des souches virales aviaires, notamment pour leur capacité à reconnaître des souches virales H5N1 ou H7N1.

En définitive on sélectionne les anticorps monoclonaux qui reconnaissent à la fois des souches de type A appartenant au sous types H1N1, des souches appartenant au sous type H3N2, et des souches de virus de type B. De façon préférée, on sélectionne ceux qui reconnaissent à la fois des souches de virus de type B appartenant au groupe B/victoria et au groupe B/Yagamata. C'est le cas notamment des anticorps monoclonal Y13F9 et Y6F5 qui sont décrits dans l'exemple 1.

Pour la caractérisation des anticorps monoclonaux, on utilise des méthodes bien connues de l'homme du métier, telles que l'ELISA, le western blotting, le dot blotting ou la résonance plasmonique de surface.

La présente invention englobe également les fragments et dérivés des anticorps monoclonaux de l'invention, en particulier les fragments Fab, Fab', F(ab)'₂ et sCFv (Blazar et al, 1997, Journal of Immunology 159 : 5821-5833 et Bird et al., 1988, Science 242 :423-426) ainsi que les conjugués. Les dérivés des anticorps monoclonaux de l'invention comprennent, entre autres, les anticorps sous une forme humanisée. Les méthodes pour produire des fragments d'anticorps monoclonaux ainsi que les dérivés d'anticorps monoclonaux, incluant notamment les anticorps monoclonaux sous une forme humanisée, sont bien connues de l'homme du métier. Les formes humanisées d'anticorps non humains, par exemple murins, sont des anticorps chimères qui comprennent une séquence minimale dérivée d'une immunoglobuline non humaine. Pour la plupart, les anticorps humanisés sont des immunoglobulines humaines (anticorps récepteur) dans lesquelles des résidus d'une région hypervariable du récepteur sont remplacés par des résidus d'une région hypervariable d'une espèce donneur (anticorps donneur) non humaine, telle que souris, rat, lapin ou primate non humain, ayant la spécificité, l'affnité et la capacité souhaitées. Généralement la région hypervariable provient de la souris. Dans certains cas, les résidus (FR) de la région Fv de l'immunoglobuline humaine sont remplacés par des résidus correspondants non humains, le plus souvent d'origine murine. De plus, les anticorps humanisés peuvent comprendre des résidus qui ne sont pas trouvés dans l'anticorps receveur ou dans l'anticorps donneur. Ces modifications sont effectuées pour améliorer les performances de l'anticorps. En général, l'anticorps humanisé comprendra au moins et de préférence deux domaines variables, dans lesquels tout ou à peu près tout des boucles hypervariables correspondent à une immunoglobuline non humaine (en général elles correspondent à une immunoglobuline murine) et tout ou à peu près tout des régions FR seront celles d'une immunoglobuline humaine. Les anticorps humanisés facultativement pourront aussi comprendre au moins une partie d'une région constante (Fc) d'une immunoglobuline, telle qu'une immunoglobuline humaine (Jones et al., Nature 321 : 522-525 (1986) ; Reichmann et al., Nature 332 : 323-329 (1988) ; et Presta et al., Curr. Op. Struct. Biol. 2 : 593-596 (1992).

Les anticorps monoclonaux de l'invention sont utilisables dans un immunoessai pour la détection et/ou la quantification de l'HA d'un virus grippal de type A ou B dans un matériel biologique d'origine aviaire présumé contenir une ou plusieurs souches de virus grippales. On peut les utiliser notamment dans le cadre de l'étiologie d'une infection virale, ou pour contrôler le contenu d'un vaccin antigrippal.

Dans le cadre du diagnostic d'une infection, le prélèvement est généralement un prélèvement de gorge, de trachée ou de sécrétions nasales. On a souvent recours à des prélèvements cloacaux ou fécaux, lorsqu'il y a une suspicion de grippe aviaire dans un élevage de volailles. On peut également faire des prélèvements de tissus. Les prélèvements sont introduits dans des milieux de transport (milieu M 199, Milieu à base de glycerol) puis généralement on amplifie le virus grippal s'il est présent dans le prélèvement d'origine en l'inoculant dans des oeufs embryonnés de poule. Après amplification du virus, récolte du liquide amniotique et centrifugation, on prélève le surnageant que l'on teste dans un immunoessai au moyen d'un anticorps monoclonal selon l'invention. On utilise de préférence la technique du western blotting lorsqu'on cherche à détecter du virus de la grippe à partir du liquide allantoïque brut.

Dans le cadre des contrôles qui sont pratiqués sur les vaccins antigrippaux, on détermine la quantité d'HA présente dans le produit fini qui est sous la forme d'un vaccin purifié, ou de pratiquer des tests de contrôles aux différentes étapes de la fabrication du vaccin (étape 6, étape 15, étape 21). Le matériel biologique peut être une préparation vaccinale purifiée ou une préparation en cours de fabrication. La préparation vaccinale purifiée peut être sous différentes formes : virus entier, atténué ou tué, sous la forme de virus splitté, de virosomes, ou même sous une forme sous unitaire contenant essentiellement une ou plusieurs HA virales hautement purifiées. Le vaccin peut être sous une forme monovalente lorsqu'il est produit à partir d'une seule souche de virus ou sous une forme multivalente lorsqu'il est produit à partir de plusieurs souches (généralement deux souches de virus de type A appartenant à des sous types différents et une souche de virus de type B). Les virus qui servent à la fabrication du vaccin sont généralement produits sur oeufs embryonnés et le matériel biologique de départ est le liquide allantoïque infecté. L'HA des souches de virus de type A qui est utilisée pour la fabrication du vaccin a le sous type H1, H3, H5 ou H7.

L'invention a donc également pour objet une méthode de détection d'un virus de la grippe ou de l'HA su virus grippal dans un matériel biologique qui comprend une étape où on met le dit matériel en contact avec un anticorps monoclonal selon l'invention ou un fragment ou un dérivé de celui ci de façon à former des complexes antigène/anticorps suite à l'interaction spécifique entre ledit anticorps monoclonal ou fragment ou dérivé de celui ci et l'hémagglutinine du virus de la grippe contenue dans ledit matériel biologique, et une étape où on met en évidence la présence de ces complexes antigène/anticorps.

Cette méthode est notamment mise en oeuvre dans les techniques ELISA direct, de dot blot ou en utilisant un appareillage type Biacore. On dépose le matériel biologique sur un support solide sur lequel s'adsorbe les protéines, comme par exemple un support à base de polystyrène, de nylon ou de nitrocellulose. Après une période d'incubation, suivie de lavages on rajoute un anticorps monoclonal selon l'invention qui est directement ou indirectement marqué. L'anticorps monoclonal se fixe spécifiquement sur les sites glycosylés des HA virales qui se sont adsorbées sur le support solide. On mesure ensuite la quantité de marqueur qui s'est fixé sur les complexes antigène/anticorps formés en utilisant des techniques classiques de l'homme du métier. On peut aussi évaluer directement l'interaction de l'antigène avec l'anticorps monoclonal durant la formation des complexes antigène/anticorps en mesurant l'intensité du signal de résonnance plasmonique en utilisant un appareillage de type Biacore.

La méthode de détection d'un virus de la grippe dans un matériel biologique d'origine aviaire, selon l'invention est également mise en oeuvre dans la technique du Western-Blotting à une ou deux dimensions. Dans ce cas, la présence des complexes antigène/anticorps formés n'est plus détectée au moyen d'un marqueur ou au moyen d'un signal de résonnance plasmonique comme dans les cas précédents mais par l'existence d'une bande électrophorétique d'un poids moléculaire d'environ 75KD ou de 50KD (dans le cas où le matériel est traité au β mercaptoethanol) qui est caractéristique de l'HA.

Lorsque le matériel biologique d'origine aviaire à analyser est enrichi en virus grippaux ou en composants de virus grippaux contenant de l'HA, et qu'il contient peu ou pas de contaminants qui peuvent donner des résultats faussement positifs dus à la présence de contaminants glycosylés dans le matériel, on utilise surtout les techniques ELISA direct, de dot blot ou l'appareillage type Biacore. On utilise ces techniques notamment pour détecter ou doser le contenu en HA dans un vaccin anti grippal purifié qui a été obtenu en utilisant des oeufs embryonnés de poule infectés par une ou plusieurs souches virales. Un vaccin antigrippal est considéré comme purifié lorsque le contenu en HA représente au moins 30% des protéines totales (p/p).

On utilise surtout les techniques de Western-blotting quand le matériel biologique est non purifié (comme par exemple du liquide allantoïque infecté) de façon à s'affranchir du problème causé éventuellement par la présence de contaminants glycosylés susceptibles d'interagir avec un anticorps monoclonal selon l'invention.

Selon un autre aspect de l'invention, la méthode mise en oeuvre comprend une étape préalable selon laquelle on met le matériel en contact avec un support solide sur lequel est fixé un agent de capture reconnaissant spécifiquement une structure du virus grippal. Le virus, la fraction de virus, ou l'HA est alors retenu ou « capturé » spécifiquement sur le support solide tandis que les autres composants du matériel biologique, sont éliminés pendant les lavages qui ont lieu après l'étape de capture. On procède ensuite comme précédemment, en utilisant un anticorps monoclonal selon l'invention pour mettre en évidence ou doser quantitativement l(les)'hémagglutinine(s) des virus grippaux présents dans le matériel biologique.

L'agent de capture est fixé sur le support solide en utilisant des moyens connus de l'homme de l'art, notamment par le biais d'interactions hydrophobes, de liaisons hydrogène ou même par le biais de liaisons covalentes.

On emploie généralement un anticorps monoclonal spécifique de la partie protéique de l'HA comme agent de capture mails on pourrait utiliser d'autres agents de capture, notamment des anticorps dirigés contre les protéines de l'enveloppe du virus (par exemple des anticorps spécifiques de la neuraminidase ou de la protéine M) ou même la fétuine.

Habituellement, on utilise comme agent de capture un anticorps monoclonal spécifique de la partie protéique de l'HA du virus grippal. On retient uniquement l'HA d'une souche virale particulière lorsque l'anticorps monoclonal a une spécificité dite de «souche». Lorsque l'anticorps monoclonal a une spécificité de « sous type » on retient sur le support les HA des souches virales qui ont le même sous type d'HA. A titre d'exemple, on cite les anticorps monoclonaux M322210 (TEBU), MAB825430-2F11-F5-A5 (CHEMICON), 9E10 (laboratoire de Nakagawa) ou 7H11 (laboratoire de Nakagawa) qui reconnaissent respectivement les sous types H1 et H3 de l'HA pour les deux premiers, tandis que les deux derniers reconnaissent respectivement les HA des souches virales de type B appartenant au groupe Victoria et les HA des souches virales de type B appartenant au groupe Yagamata. On utilise habituellement un anticorps monoclonal spécifique de la partie protéique de l'HA comme agent de capture que ce soit pour doser spécifiquement dans du matériel biologique la quantité d'HA provenant d'une souche virale particulière ou la quantité d'HA provenant d'une ou plusieurs souches appartenant à un même sous type.

C'est pourquoi l'invention a également pour objet dans un aspect particulier : Une méthode de dosage de l'hémagglutinine du virus grippal contenue dans un matériel biologique qui comprend les étapes suivantes :
a) on met le matériel en contact avec un support solide sur lequel est fixé un premier anticorps monoclonal spécifique reconnaissant une structure protéique de l'hémagglutinine du virus grippal ;
b) on retient spécifiquement sur le support solide les complexes antigène/anticorps qui résultent de l'interaction spécifique de cet anticorps monoclonal avec l'hémagglutinine du virus grippal contenue dans le matériel biologique ;
c) on met en contact les complexes antigène/anticorps retenus sur le support solide avec un deuxième anticorps monoclonal selon l'invention ou un fragment ou un dérivé de celui-ci et portant directement ou indirectement un marqueur ;
d) on mesure la quantité de marqueur qui s'est fixé spécifiquement sur les complexes antigène/anticorps ; et
e) à partir de la quantité de marqueur mesurée, on détermine la quantité d'hémagglutinine contenue dans le matériel par comparaison avec un ou plusieurs réactifs de référence.

Le deuxième anticorps monoclonal selon l'invention (ou anticorps monoclonal de révélation) est directement marqué lorsqu'il a le même isotype et qu'il provient de la même espèce animale que le premier anticorps monoclonal (ou anticorps monoclonal de capture). Dans les autres cas, le deuxième anticorps monoclonal peut être directement ou indirectement marqué. Dans le cas d'un marquage indirect, le deuxième anticorps monoclonal selon l'invention est reconnu par l'intermédiaire d'un troisième anticorps directement marqué spécifique de l'isotype ou de l'espèce animale du deuxième anticorps.

Dans un mode de réalisation particulier, la méthode de dosage selon l'invention s'applique à du matériel biologique représenté par une préparation vaccinale contenant de l'HA provenant d'une ou plusieurs souches de virus grippaux différents. Cette préparation vaccinale peut se présenter sous différentes formes (pourvu qu'elle contienne de l'HA) ; elle peut être sous forme de vaccin purifié ou de matériel récolté aux différentes étapes de fabrication du vaccin, incluant notamment le liquide allantoïque brut infecté obtenu à partir d'oeufs embryonnés de poules infectées.

A titre d'exemple, on applique les techniques immunoenzymatiques de « type sandwich » les techniques radio-immunologiques. immunonéphélométriques, ou d'immunofluorescence indirecte bien connues de l'homme du métier pour mettre en oeuvre cette méthode de dosage. Au lieu de mesurer la quantité de marqueur, on peut, de façon similaire, évaluer directement l'interaction du complexe antigène-anticorps à l'aide du système Biacore.

Lorsque le matériel biologique contient peu ou pas de contaminants glycosylés susceptibles d'être reconnus par un anticorps monoclonal selon l'invention, comme dans le cas des préparations vaccinales purifiées, l'agent de capture peut être représenté par un anticorps monoclonal selon l'invention tel que l'anticorps monoclonal Y6F5 ou l'anticorps monoclonal Y13F9. Un seul et même anticorps monoclonal selon l'invention peut être utilisé à la fois comme agent de capture et anticorps de révélation. On peut également associer deux anticorps monoclonaux différents, en utilisant par exemple l'anticorps monoclonal Y13F9 comme anticorps de capture et l'anticorps monoclonal Y6F5 comme anticorps de révélation.

C'est pourquoi, dans un aspect particulier, l'invention a également pour objet : Une méthode de dosage de l'hémagglutinine d'un virus grippal contenue dans un matériel biologique qui comprend les étapes suivantes :
a) on met le matériel en contact avec un support solide sur lequel est fixé un premier anticorps monoclonal selon l'invention ou un fragment ou un dérivé de celui-ci ;
b) on retient spécifiquement sur le support solide les complexes antigène-anticorps qui résultent de l'interaction spécifique de l'anticorps monoclonal avec l'hémagglutinine du virus grippal contenue dans le matériel biologique;
c) on met en contact les complexes antigène/anticorps retenus sur le support solide avec un deuxième anticorps monoclonal selon l'invention ou un fragment ou un dérivé de celui-ci portant directement ou indirectement un marqueur, le deuxième anticorps monoclonal pouvant être distinct ou identique au premier anticorps;
d) on mesure la quantité de marqueur qui s'est fixé sur les complexes antigène/anticorps ; et
e) à partir de la quantité de marqueur mesurée, on détermine la quantité d'hémagglutinine contenue dans le matériel par comparaison avec un ou plusieurs réactifs de référence.

Comme indiqué précédemment, le deuxième anticorps monoclonal (ou anticorps monoclonal de révélation) est directement marqué lorsqu'il a le même isotype et qu'il provient de la même espèce animale que le premier anticorps monoclonal (ou anticorps monoclonal de capture). Dans les autres cas, le deuxième anticorps monoclonal peut être directement ou indirectement marqué (dans ce cas le deuxième anticorps monoclonal est marqué par l'intermédiaire d'un troisième anticorps directement marqué reconnaissant l'isotype ou la spécificité d'espèce du deuxième anticorps). En général, par souci de simplification de la méthode de dosage selon l'invention, le deuxième anticorps monoclonal est habituellement directement marqué.

Dans un mode de réalisation particulier, le matériel biologique est représenté par une préparation vaccinale purifiée contenant de l'HA provenant d'une ou plusieurs souches de virus grippaux. Comme indiqué précédemment, cette préparation vaccinale purifiée peut se présenter sous différentes formes pourvue qu'elle contienne de l'HA du virus grippal. Elle peut notamment contenir des souches de virus grippaux entières (qui peuvent être aussi inactivées) ou splittées, ou sous forme de virosomes ou sous forme de vaccin sous unitaire contenant essentiellement une ou plusieurs HA hautement purifiées

Dans un autre aspect, l'invention a pour objet :
Un procédé de purification du virus de la grippe ou de l'hémagglutinine du virus de la grippe à partir d'un matériel biologique qui comprend les étapes suivantes:
   a) on met le matériel en contact avec un support solide sur lequel est fixé un premier anticorps monoclonal selon l'invention ou un fragment ou un dérivé de celui-ci;
   b) on retient spécifiquement sur le support solide les complexes antigène/anticorps qui résultent de l'interaction spécifique de l'anticorps monoclonal avec l'hémagglutinine du virus grippal contenue dans le matériel;
   c) on libère le virus de la grippe ou l'hémagglutinine du virus grippal des complexes antigène-anticorps retenus sur le support solide ; et
   d) on récupère le virus de la grippe ou l'hémagglutinine du virus grippal.

Habituellement, le support solide est un support chromatographique à base de dextran, d'agarose ou de silice. L'anticorps monoclonal est habituellement lié de façon covalente au support chromatographique en mettant en oeuvre des méthodes de couplage bien connues de l'homme du métier.

On met en contact l'échantillon à purifier avec le support immunochromatographique qui retient sélectivement l'HA du virus grippal ou le virus grippal. Les composants non reconnus par l'anticorps monoclonal selon l'invention (contaminants) ne sont pas retenus sur le support solide et sont éliminés directement dans un tampon chromatographique. On libère ensuite le virus grippal ou l'hémagglutinine du virus grippal du support solide en utilisant des tampons d'élutions spécifiques tels que le tampon glycine à pH= 2, des agents chaotropiques de forte molarité en sels. On recueille le pic chromatographique correspondant à l'HA ou au virus grippal que l'on dialyse généralement contre un tampon physiologique. On peut augmenter par la suite le degré de pureté de la préparation en combinant ce procédé de purification avec d'autre procédés chromatographiques, comme la chromatographie d'exclusion ou la chromatographie échangeuse d'ions.

L'invention concerne aussi une composition pharmaceutique comprenant un ou plusieurs anticorps monoclonaux selon l'invention ou des dérivés ou fragments de ceux-ci en mélange avec un véhicule pharmaceutiquement acceptable. Par véhicule pharmaceutiquement acceptable on entend les supports et véhicules administrables à l'être humain ou à un animal, tels que décrits par exemple dans Remington's Pharmaceutical Sciences 16"'ed., Mack Publishing Co.

Elle concerne également l'utilisation d'un ou plusieurs anticorps monoclonaux ou des dérivés ou fragments de ceux-ci selon l'invention pour la préparation d'un médicament pour traiter ou prévenir une infection grippale par immunisation passive.

Elle concerne une méthode pour prévenir ou traiter un individu contre la grippe, selon laquelle on administre à l'individu une quantité effective d'une composition pharmaceutique comprenant un ou plusieurs anticorps monoclonaux selon l'invention.

L'invention comprend donc une méthode d'immunisation passive à but thérapeutique ou prophylactique contre la grippe qui consiste à administrer à un individu, notamment chez l'homme, chez des mammifères (tels que le chien, le chat, le porc, le cheval) ou à des oiseaux, une quantité thérapeutiquement ou prophylactiquement efficace d'un anticorps monoclonal, fragment ou dérivé de l'invention ou leurs combinaisons. Un ou plusieurs autres anticorps monoclonaux ou polyclonaux dirigés contre une structure antigénique située sur la partie protéique de l'HA ou dirigés contre la NA peuvent également être associés ou combinés à un ou plusieurs anticorps monoclonaux selon l'invention.

Les exemples qui suivent illustrent de façon non limitative différents modes de réalisation de l'invention.

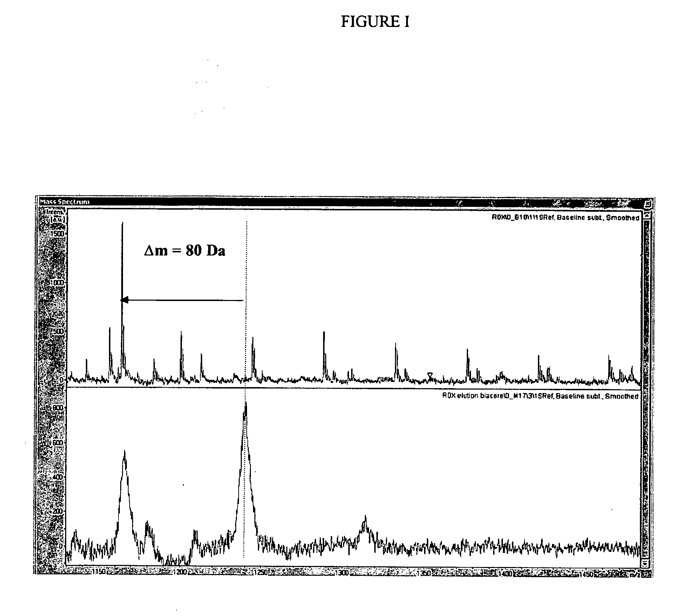

La figure 1 représente les spectres de masse MALDI-TOF des glycanes avant (spectre du bas) et après traitement à l'ammoniaque (spectre du haut).

### Exemples

### Exemple 1 : obtention d'anticorps monoclonaux selon l'invention

### 1.1 Production d'anticorps monoclonaux

Des souris femelles BALB/c, de 6 à 8 semaines ont été immunisées selon le protocole suivant : elles ont reçu une première injection intrapéritonéale de 100µl d'un mélange d'HA et de NA purifiées ayant un titre hémagglutinant de 64000 unités sous la forme d'une émulsion obtenue avec un volume égal d'adjuvant complet de Freund suivie de deux injections à 2 semaines d'intervalle du même mélange sous la forme d'une émulsion réalisée en présence d'adjuvant incomplet de Freund. Le mélange d'HA et de NA purifiées a été préparé à partir de la souche A/Sydney/5/97 (H3N2) IVR 108. La souche virale a été produite sur oeufs embryonnés et purifiée en filtrant le liquide allantoïque infecté sous 0,45µ puis en réalisant une sédimentation sur un gradient de saccharose. On a ensuite traité la souche virale purifiée par le triton X100 selon la technique de Gerentes et coll. (journal of Virological methods : 73 (1998) 185-195) pour obtenir le mélange d'HA et de NA. 3 jours après la dernière injection, les cellules de la rate ont été prélevées et fusionnées avec la lignée cellulaire de myélome de souris p3x63-Ag8.653, selon la technique de Kohler et Milstein. Les surnageants d'hybridomes ont été criblés en utilisant des tests ELISA dans lequel l'antigène de « coating » était soit le virus purifié, soit la NA qui a été purifiée par immunochromatographie à partir du mélange d'HA et de NA selon la technique de Gerentes et coll. (Journal of Virological methods : 58 (1996) 155-165. Les hybridomes qui ont été positifs dans le test ELISA utilisant comme antigène de « coating » le virus purifié et qui ont été négatifs dans le test ELISA utilisant comme antigène de « coating » la NA purifiée ont été ensuite clonés à plusieurs reprises puis produits sous forme d'ascites. Les ascites ont été obtenus à partir de souris, ayant reçu au préalable une injection de Pristane (nom commercial) suivi à quelques jours d'intervalle d'une injection d'environ 10⁶ cellules d'hybridomes. Les anticorps monoclonaux produits par ces hybridomes ont été utilisés par la suite sous la forme de liquides d'ascites ou sous forme de liquides d'ascites purifiés

Les liquides d'ascites ont été purifiés par centrifugation à 10 000g pendant 5 minutes à +4°C. Les surnageants ont ensuite été traités au sulfate d'ammonium saturé (concentration finale 40% du volume final) pendant 5 min sous agitation pour faire précipiter les anticorps monoclonaux. Les précipités ont été recueillis par centrifugation à 3500g pendant 15 min. Les culots ont été repris dans 2 ml d'un tampon phosphate 8mM, pH=7,5 (PBS 1x) sans Ca²⁺ ni Mg²⁺ puis dialysés contre 4 litres de PBS 1x sans Ca²⁺ ni Mg²⁺ pendant 2 fois 2 h avec changement du PBS au bout de 2h. Les anticorps monoclonaux dialysés ont ensuite été centrifugés pendant 10 min à 10 000g puis dosés par la méthode de Bradford.

Pour leur utilisation dans des tests de contrôles d'échantillons biologiques, notamment pour le contrôle des vaccins grippaux, on a marqué à la peroxydase les anticorps monoclonaux les plus intéressants, comme l'anticorps monoclonal Y13F9 en utilisant la méthode de Pierce.

### 1.2 Etude de la spécificité des anticorps monoclonaux

On a testé les anticorps monoclonaux obtenus selon le protocole décrit dans le paragraphe 1.1 ainsi qu'un certain nombre d'anticorps monoclonaux provenant du commerce ou du laboratoire de Naoko Nakagawa (anticorps spécifiques des souches virales de type B) pour leur capacité à reconnaître les hémaglutinines des souches A/New Caledonia/20/99 (H1N1), A/Panama/2007/99 (H3N2) et B/Shandong/7/97 (souches du vaccin trivalent de l'année 2003-2004). Ces souches virales ont été produites sur oeuf embryonnés puis purifiées sur gradient de saccharose. On a déterminé la quantité d'HA contenue dans chaque souche purifiée en utilisant le test officielle SRD (Single Radial Immunodiffusion). Le contenu en HA représente au moins 30% des protéines virales. 3 séries de microplaques ELISA (Dynex ® ref : 655071) ont été « coatées » avec l'une des 3 souches monovalentes purifiées en introduisant dans chaque puits 100µl d'une souche monovalente purifiée contenant 1µg/ml d'HA en tampon carbonate 0,05M, pH : 9,6 pendant une nuit à +4°C. Après aspiration du contenu des microplaques, les microplaques ont ensuite été saturées pendant 1 heure à 37°C avec 150µl/puits d'un tampon phosphate-0,05% tween 20-1% de lait écrémé (tampon de saturation). Après aspiration du contenu, on a introduit dans chaque puits des dilutions successives de raison 2 de chaque anticorps monoclonal purifié ou sous forme de liquide d'ascite, la première dilution testée étant 1/1000. Les dilutions ont été réalisées en tampon de saturation. On a introduit comme contrôles positifs des sérums de souris hyperimmunisées dilués au 1/10000. Après une incubation d'1,5 heures à 37°C, suivie de 4 lavages en tampon phosphate-0,05% tween 20 (tampon de lavage) on a introduit dans chaque puits 100µl d'un conjugué de mouton anti 1g de souris marqué à la peroxydase (Amersham NA931) dilué au 1/4000 en tampon phosphate-0,05% tween 20. Après une nouvelle incubation d'1,5 heure à 37°C, suivie de 4 lavages, la réaction enzymatique a été révélée avec une solution de tétraméthylbenzidine (TMB) ; l'intensité de la coloration des puits a été mesurée par spectrophotométrie à 450 et à 600nm. On a déterminé ainsi pour chaque anticorps monoclonal testé un titre qui est l'inverse de la dilution de l'anticorps (exprimé en log₁₀) qui donnait une densité optique (D.O) de 1 à 450 nm. Un titre inférieur ou égal à 2,7, symbolisé dans le tableau 1 sous la forme (≤) signifie que l'anticorps monoclonal ne reconnait pas l'HA de la souche purifiée monovalente. On a identifié 2 anticorps monoclonaux produits par les hybridomes Y13F9 et Y6F5 qui ont été obtenus selon le protocole de l'exemple 1 qui reconnaissent les HA des 3 souches purifiées: A/New Caledonia/20/99 (H1N1), A/Panama/2007/99 (H3N2) et B/Shandong/7/97 (titre >2,7 dans les 3 tests ELISA). Par contre, aucun des anticorps monoclonaux du commerce (1351, 48079, M322210, 58CE8-1-5, 58AB7-19-18, 621D5-11, 30-2F11-F7-A5, BGN/5G8, C102, 3B3, 4H7) ou ceux du laboratoire de Naoko Nakagawa (1H12, 2H12, 5B12, 9E10, 10B8) (qui a obtenu une batterie d'anticorps monoclonaux contre les souches virales de type B (Journal of Virological methods 79 : 113-120 (1999) ; Journal of Medical Virology 65 :745-750 (2001) ; Journal of General Virology 82 : 2169-2172)), reconnaissent les HA des 3 souches. L'anticorps monoclonal 30-2F11-F7-A5 reconnait les HA des souches A/New Caledonia/20/99 (H1N1) et A/Panama/2007/99 (H3N2). Les autres anticorps monoclonaux ne reconnaissent qu'une seule des 3 souches testées (cf. tableau 1)

**Tableau 1 : Reconnaissance des souches A/NC/20/99 (H1N1), A/Panama/2007/99 (H3N2) et B/shangdong/7/97 par différents anticorps monoclonaux dirigés contre l'HA du virus grippal.**

| Nom de l'hybridome | isotype | Spécificité «d'origine » | A/New Caledonia/20/99 (H1N1) | A/Panama/2007/99 (H3N2) | B/Shandong/7/97 (B) |
|---|---|---|---|---|---|
| Y13F9 | IgM | A/Sydney/5/97 (H3N2) | 5,25* | 5,28 | 5,3 |
| Y6F5 | IgM | A/Sydney/5/97 (H3N2) | 3,9 | 3,97 | 4,0 |
| 1351 | IgG1 | H1N1 | 3,6 | ≤ | ≤ |
| 48079 | IgG1 | H1N1 | 5,65 | ≤ | |
| M322210 | IgG1 | H1N1 | 5,80 | ≤ | ≤ |
| 58CE8-1-5 | | A/Taiwan/1/86 (H1N1) | ≤ | | |
| 58AB7-19-18 | IgG1 | H1N1 | ≤ | 3,0 | ≤ |
| 62ID5-11 | | A/Shang./11/97 (H3N2) | | ≤ | |
| 30-2F11-F7-A5 | IgG2a | H3N2 | 2,85 | 5,6 | ≤ |
| BGN/5G8 | | B/Panama/45/90 | ≤ | ≤ | ≤ |
| C102 | IgG1 | H1N1 | 5,8 | ≤ | ≤ |
| 3B3 | | H1N1 | 5,25 | ≤ | NS |
| 4H7 | IgG1 | B/Panama/45/90 | ≤ | ≤ | ≤ |
| 1H12 | IgG1 | B/Nagasaki/1/87 | ≤ | ≤ | 5,1 |
| 2H12 | IgG3 | B/Nagasaki/1/87 | ≤ | ≤ | 4,8 |
| 5B12 | | B/Nagasaki/1/87 | ≤ | ≤ | 3,1 |
| 9E1O | IgG1 | B/Nagasaki/1/87 | ≤ | ≤ | 6,1 |
| 10B8 | IgG2a | B/Nagasaki/1/87 | ≤ | ≤ | >6,1 |

| | | | | | |
|---|---|---|---|---|---|
| * : titre ELISA NS : non spécifique | | | | | |

Les anticorps monoclonaux Y13F9 et Y6F5 ont été testés également pour leur capacité à reconnaître d'autres souches de virus grippaux, notamment les souches A/Taïwan/1/86xX31 (H1N1), A/Chile/1/83X83 (H1N1), A/Wisconsin/67/2005 (H3N2), A/New York/55/2004 (H3N2). A/Wyoming/03/2003 (H3N2), A/Honk Kong/1/68 (H3N2), A/Mississipi/1/85X87 (H3N2), A/Sichuan/2/87 (H3N2), A/Leningrad/360/86 (H3N2), A/Shanghai/11/87X99, B/Malaysia/2506/04, B/Jiangsu/10/2003, B/Brisbane/32/02, B/Yagamata/16/88, B/Beijing/1/87. Toutes ces souches ont été produites sur oeufs embryonnés et purifiées et étaient sous forme de virus purifiés, ou de virus splittés lorsque le virus purifié a subi un traitement au tween-ether ou au triton. Dans certains cas les souches testées étaient sous forme de vaccin monovalent ou de mélange de vaccins monovalents On a utilisé une technique ELISA indirect « type sandwich » dans laquelle l'HA des souches de virus testées est prise en sandwich entre l'anticorps monoclonal Y13F9 ou Y6F5 qui a servi d'anticorps de capture et l'anticorps monoclonal Y13F9 directement marqué à la peroxydase qui a servi d'anticorps de révélation. Pour la mise en oeuvre de la technique, les plaques ELISA ont été « coatées » avec 100µl d'une solution d'anticorps monoclonal purifié (utilisé comme anticorps de capture) à une concentration d'environ 2µg/ml en tampon carbonate pH=9,6 pendant une nuit à +4°C suivie d'une étape de saturation des plaques comme précédemment. On a ensuite déposé 100µl d'un échantillon de la souche à tester en réalisant des dilutions successives de l'échantillon dans un tampon de saturation puis on a laissé reposer les microplaques pendant 1,5 heure à 37°C. Après lavage des plaques, on a déposé 100µl d'une solution d'un anticorps de révélation (anticorps monoclonal Y13F9 directement marqué à la peroxydase) à la concentration de 0,1 µg/ml en tampon de saturation. Après une nouvelle incubation d'1,5 heure à 37°C, suivie de lavages, les étapes de révélation et de mesure de l'intensité de la réaction ont été réalisées comme précédemment. Les résultats ont révélé que toutes les souches H1N1, H3N2 et les souches de virus B testées étaient reconnues aussi bien par l'anticorps monoclonal Y6F5 que l'anticorps monoclonal Y13F9. Ces deux anticorps monoclonaux inhibaient également l'activité hémagglutinante des souches de virus A/beijing/32/92 (H3N2), A/Shangdong/9/93 (H3N2), A/Johannesburg/33/94, (H3N2), A/Nanchang/933/95 (H3N2), A/Sydney/5/97(H3N2). Enfin la souche A/Vietnam/1194/04 qui est une souche aviaire de type H5N1 est reconnue par l'anticorps monoclonal Y13F9.

Ces résultats prouvent bien que les anticorps monoclonaux Y6F5 et Y13F9 reconnaissent aussi bien les hémagglutinines H3 et H1 des virus grippaux de type A puisque de nombreuses souches H3N2 et H1N1 sont reconnues mais également les hémagglutinines des virus grippaux de type B puisque de nombreuses souches de virus grippaux de type B appartenant aussi bien au groupe B/Yagamata que B/Victoria sont reconnues. L'anticorps monoclonal Y13F9 reconnait également l'hémagglutinine H5.

### 1.3 Utilisation des anticorps monoclonaux pour le contrôle des vaccins anti-grippaux

Nous avons utilisé deux méthodes ELISA indirectes (type sandwich)

Dans la première méthode, l'anticorps monoclonal de capture était un anticorps monoclonal spécifique de « sous type » ou un anticorps monoclonal spécifique des virus de type B, l'anticorps monoclonal de révélation étant l'anticorps monoclonal Y13F9 marqué à la peroxydase. Ce type d'ELISA, spécifique de « sous type » ou spécifique des virus de type B, a permis de doser le contenu en HA de chacune des souches présentes dans un vaccin trivalent. Il contenait en effet deux souches de virus de type A appartenant à des sous types distincts et une souche de virus de type B. On a dosé la quantité d'HA de chacune des souches vaccinales contenue dans le vaccin trivalent en mettant en oeuvre 3 ELISA différents, spécifiques de « souche » se distinguant uniquement par l'anticorps monoclonal de capture. Par addition des 3 quantités, on a déterminé la quantité totale d'HA contenue dans le vaccin trivalent.

Dans la deuxième méthode, on a utilisé un seul et même anticorps monoclonal selon l'invention servant à la fois comme anticorps de capture et comme anticorps de révélation. On a utilisé l'anticorps monoclonal Y13F9 comme anticorps de capture et l'anticorps monoclonal Y13F9 marqué à la peroxydase comme anticorps de révélation. On a déterminé la quantité d'HA totale contenu dans le vaccin trivalent en mettant en oeuvre un seul ELISA mais dans ce cas on ne pouvait pas doser la quantité de HA qui « revenait » à chacune des souches. Cette méthode de dosage est considérée comme « polyspécifique » car on peut contrôler la quantité d'HA contenue dans des vaccins anti-grippaux d'années différentes.

On a utilisé ces deux méthodes dans le cadre des contrôles effectués sur vaccin de l'année 2003-2004 qui contenait les souches A/New Caledonia/20/99 (H1N1), A/Panama/2007/99 (H3N2) et B/Shandong/7/97. Le vaccin a été contrôlé à différentes étapes de sa fabrication : au début de sa fabrication, c'est-à-dire au stade où le vaccin était représenté par le liquide allantoïque brut infecté (étape 6). au stade où le vaccin était sous forme de virus entier concentré et purifié (étape 15), et au stade où le vaccin était sous forme de vaccin trivalent (vaxigrip) (vaccin contenant les 3 souches de virus purifiés et splittés sous l'action de détergent). Dans le cadre de ces contrôles on a également utilisé les antigènes de référence fournis par le NISBC qui étaient des solutions de virus semi-purifiés provenant de la souche A/New Caledonia/20/99 (lot 01/614), de la souche A/Panama/2007/99 (lot 02/100) ou de la souche B/Shandong/7/97 (lot 02/108) et qui titraient théoriquement 65, 53 et 55 µg/ml d'HA selon la méthode de référence SRD. On a également fait des contrôles sur l'HA purifiée extrait de chacune des 3 souches de virus concentrés et purifiés (étape 15) par un traitement à la bromélaïne suivi d'une chromatographie par exclusion (HA purifiée). Le protocole suivi pour l'extraction et la purification de l'HA était celui décrit dans le paragraphe 2.1.

Pour la mise en oeuvre des 3 ELISA spécifiques de la première méthode on a utilisé comme anticorps monoclonal de capture une préparation purifiée d'anticorps monoclonal M322210 (Fitzgerald-ref 10150) pour doser le contenu en HA de la souche A/NC/20/99 (H1N1), une préparation purifiée d'anticorps monoclonal MAB825430-2F11-F7-A5 (Chemicon-ref mab8254) pour doser le contenu en HA de la souche A/Panama/2007/99 (H3N2) et une préparation d'anticorps monoclonal 9E10 pour la souche B/Shangdong/7/97. Dilués en tampon Carbonate/Bicarbonate 0.05M pH 9.6, ils ont été déposés au 1/100^{ième}, au fond des puits d'une plaque 96 puits (Dynex) à raison de 100 µl par puits. Après une nuit à 4°C, la plaque a été vidée puis saturée avec 150 µl par puits de tampon Phosphate-Tween 20 (0.05%) contenant 1% de lait écrémé. Après 1 h 30 d'incubation à 37°C, la plaque a été à nouveau vidée, puis on a déposé, par puits, 100 µl de l'échantillon à doser traité ou non par le zwittergent. Les échantillons à doser ont été conservés à -80°C et soniqués après décongélation avant emploi. Par ailleurs, si les échantillons à contrôler contenaient du virus non splitté (antigènes du NIBSC, étape 6 ou étape 15), ils ont été préalablement mis en contact pendant 30 min. à la température du laboratoire sous agitation avec une solution de zwittergent à 10 % (P/V) à raison de 9 volumes d'échantillon pour 1 volume de la solution de zwittergent Si la concentration de l'échantillon était connue en SRD, il a été dilué (dans le tampon de saturation), afin de déposer, dans le 1^{er} puits, 1 µg/ml de HA, puis l'échantillon a été ensuite dilué de 2 en 2 sur 12 cupules. C'est ce qui a été fait, par exemple, pour la référence et le contrôle. Si la concentration de l'échantillon à titrer était inconnu. il a été déposé pur dans le 1^{er} puits puis dilué de 2 en 2 sur 12 puits. La plaque a ensuite été incubée 1h30 à 37°C, vidée, lavée 4 fois en tampon Phosphate-Tween 20 (0.05%), puis 100 µl d'anticorps monoclonal Y13F9 conjugué à la peroxydase et dilué au 1/10 000^{ième} dans le tampon de saturation ont été ajoutés dans chaque puits. La plaque a été incubée à nouveau 1h30 à 37°C, puis vidée de son contenu, lavée 4 fois et le « sandwich » révélé par 100 µl de 3,3',5,5' tétramethylbenzidine (TMB, Tebu Bio). Après 20 min. la réaction a été stoppée par 100 µl d'HCL 1N (Prolabo). Les plaques ont été lues à 450-650 nm (Molecular Devices Versa Max).

Une référence et un contrôle, qui correspondaient à un vaccin monovalent (c.a.d à une souche de virus purifié et splitté sous l'action de détergent) dosés en SRD ont été déposés pour chaque souche, sur chaque plaque. On a établi ainsi une courbe en 4 paramètres ce qui a permis de quantifier l'HA dans les échantillons à contrôler (logiciel Soft Max Pro).

En utilisant cette méthode de dosage ELISA, on a plus la nécessité d'utiliser les réactifs du NISBC comme antigènes de référence. On peut utiliser comme référence un monovalent spécifique de souche dosé en ELISA contre de l'HA purifiée, dosée elle-même par la méthode de Bradford. Dans ce cas il n'est plus nécessaire d'attendre le dosage SRD du monovalent avant de l'utiliser comme référence.

La quantité de HA de chaque échantillon a été mesurée en µg/ml en utilisant le logiciel Soft Max Pro entre les valeurs de densité optique (DO) 0.2 et 2.0, puis calculée à partir de la courbe obtenue avec le monovalent de référence.

Le protocole opératoire utilisé pour l'ELISA polyspécifque était le même que celui qui vient d'être décrit avec les particularités suivantes. L'anticorps de capture était une solution purifiée d'anticorps monoclonal Y13F9 utilisée à une concentration de 2 µg/ml en tampon carbonate pH=9,6. Les échantillons à contrôler qui contenaient du virus non splitté (antigènes du NIBSC, ou étape 15) n'ont pas été préalablement traités au zwittergent.

Les titres ELISA spécifiques ont été comparés aux titres SRD quand ceux-ci étaient disponibles ou au dosage en Bradford lorsqu'il s'agissait de l'HA purifiée. Les résultats obtenus en utilisant les méthodes ELISA spécifiques de souches sont rassemblés dans le tableau 2. Les résultats obtenus en utilisant la méthode ELISA polyspécifique sont rassemblés dans le tableau 3.

**Tableau 2 : Dosage de l'HA de la souche A/NC/20/99 (H1N1), de l'HA de la souche A/Panama/2007/99 (H3N2) ainsi que de l'HA de la souche B/shangdong/7/97 dans différents échantillons de grippe (ELISA spécifique)**

| **Souche** | **Nature de l'échantillon** | **Titre ELISA (µg/ml de HA)** | **Titre SRD ou Bradford (µg/ml de HA)** |
|---|---|---|---|
| **A/NC/20/99 (H1N1)** | **Ag du NIBSC*** | 12.46 | 65 (SRD) |
| | **Etape 6*** | 2.6 ± 0.31 | *NA* |
| | **Etape 15*** | 104.46 | *NA* |
| | **HA purifiée** | 1025.49 | 747 (Bradford) |
| | **Vaxigrip** | 25.31 ± 13.1 | 30 (SRD) |
| **A/Panama/2007/99 (H3N2)** | **Ag du NIBSC*** | 14.52 | 53 (SRD) |
| | **Etape 6*** | 2.78 ± 1.47 | *NA* |
| | **Etape 15*** | 300.1 | *NA* |
| | **HA purifiée** | 108.34 | 318 (Bradford) |
| | **Vaxigrip** | 48.53 ± 10.38 | 30 (SRD) |
| **B/Shangdong/7/97** | **Ag du NIBSC*** | 26.24 | 55 (SRD) |
| | **Etape 6*** | 10.07 ± 5.32 | *NA* |
| | **Etape 15*** | 114.01 | *NA* |
| | **HA purifiée** | 1289.52 | 2032 (Bradford) |
| | **Vaxigrip** | 60.6 ± 16.35 | 30 (SRD) |

| | | | |
|---|---|---|---|
| *: indique que l'échantillon a été traité au zwittergent NA : Non applicable | | | |

Les échantillons qui ont été titrés 2 voire 3 fois, ont des titres ELISA qui correspondent alors à des valeurs moyennes ± l'écart type. Les échantillons qui ont été titrés qu'une fois ont qu'une seule valeur indiquée). Le titre SRD peut varier d'environ 20%. La limite de détection est de l'ordre de 1ng/ml

**Tableau 3 : Dosage de l'HA de la souche A/NC/20/99 (H1N1), de l'HA de la souche A/Panama/2007/99 (H3N2) ainsi que de l'HA de la souche B/shangdong/7/97 dans différents échantillons de grippe (ELISA polyspécifique)**

| **Souche** | **Nature de l'Antigène** | **Titre ELISA (µg/ml de HA)** | **Titre SRD ou Bradford (µg/ml de HA)** |
|---|---|---|---|
| **A/NC/20/99 (HN1)** | **Ag du NIBSC** | 65.92 ± 31.57 | 65 (SRD) |
| | **Etape 15** | 415.33 ± 51.83 | *NA* |
| | **HA purifiée** | 628.34 ± 60.14 | 747 (Bradford) |
| | **Vaxigrip** | 65.42 ± 5.04* | 90 (SRD) |
| **A/Panama/2007/99 (H3N2)** | **Ag du NIBSC** | 36.58 ± 18.32 | 53 (SRD) |
| | **Etape 15** | 158.20 | *NA* |
| | **HA purifiée** | 238.22 ± 11.89 | 318 (Bradford) |
| | **Vaxigrip** | 108.94 ± 23.13* | 90 (SRD) |
| **B/Shangdong/7/97** | **Ag du NIBSC** | 79.93 ± 22.67 | 55 (SRD) |
| | **Etape 15** | 594.88 ± 114.78 | *NA* |
| | **HA purifiée** | 1442.77 ± 44.80 | 2082 (Bradford) |
| | **Vaxigrip** | 214.15 ± 34.73* | 90 (SRD) |

| | | | |
|---|---|---|---|
| NA : non applicable * : les valeurs obtenues ne sont pas équivalentes car les références utilisées étaient différentes dans les 3 dosages. Dans le premier dosage, la référence était la souche monovalente A/NC/20/99 purifiée et splittée, dans le deuxième dosage la référence était la souche monovalente A/Panama/2007/99 purifiée et splittée et dans le troisième dosage la référence était la souche monovalente B/Shangdong/7/97 purifiée et splittée. | | | |

Les échantillons qui ont été titrés 2 voire 3 fois, ont des titres ELISA qui correspondent alors à des valeurs moyennes ± l'écart type. Les échantillons qui ont été titrés qu'une fois ont qu'une seule valeur indiquée). Le titre SRD peut varier d'environ 20%. La limite de détection est de l'ordre de 1 ng/ml

De façon générale, on observe une bonne correspondance entre les titres obtenus par la méthode SRD ou la méthode de Bradford et les titres obtenus avec les ELISA spécifiques ou avec la méthode ELISA polyspécifique Vis-à-vis des antigènes de référence du NISBC on note une meilleure correspondance entre les titres ELISA et les titres SRD lorsqu'on utilise la méthode ELISA polyspécifique. Vis-à-vis de l'HA hautement purifiée, quelque soit l'origine de la souche on note également une meilleure correspondance entre les titres ELISA et les titres obtenus par la méthode de bradford lorsqu'on utilise la méthode ELISA polyspécifique.

Les ELISA spécifiques présentent l'intérêt de pouvoir quantifier chaque souche en mélange dans un trivalent, ainsi que de procéder à la recherche d'éventuelles contaminations hétérologues mais il faut pouvoir disposer d'anticorps monoclonaux de capture spécifiques des souches vaccinales de l'année.

L'ELISA « polyspécifique » présente l'intérêt de pouvoir quantifier toute souche de virus grippal purifiée en se soustrayant aux aléas d'approvisionnement récurrent en anticorps spécifiques.

Les deux méthodes, en particulier celle qui requiert l'usage des ELISA spécifiques permettent d'envisager des contrôles de production en cours et surtout la formulation à risque du vaccin trivalent Vaxigrip à partir des vracs de chaque monovalent; indépendamment de la mise à disposition des réactifs du NIBSC.

### Exemple 2: Caractérisation de la structure antigénique reconnu par les anticorps monoclonaux selon l'invention

### 2.1 : la structure antigénique reconnue par l'anticorps monoclonal est un motif glycosylé

La souche A/New Caledonia/20/99 (H1N1) purifiée selon le protocole opératoire de l'exemple 1 a été inactivée pendant une nuit à + 4°C par une solution de β propiolactone diluée au 1/1000^{ème} puis ultracentrifugée. Le culot viral a ensuite subi un double traitement à la bromélaïne en ajoutant 55µg de Bromélaïne par mg de virus purifié en suspension dans le PBS puis en ajoutant 8µl de β mercaptoéthanol par ml de mélange pendant une nuit à +4.°C. On a extrait ainsi l'HA de l'enveloppe virale. Le surnageant a ensuite été soumis à une chromatographie d'exclusion sur gel de superose 6 (Pharmacia) dans un tampon PBS 8mM pour isoler et recueillir l'éluat contenant l'HA. Une partie a ensuite été traitée à la PNGase F en ajoutant à la solution d'HA (pH = 7,5) 1,3 unités de PNGase (Calbiochem ref: 362185.) par mg de HA. La PNGase est une endoglycosidase clivant les N-glycanes au niveau des sites asparagine. Après un traitement de 2 heures à 37°C, on a obtenu une HA déglycosylée. L'autre partie n'a pas été traitée à la PNGase. Les échantillons de HA traités et non traités à la PNGase F ont été contrôlés par Western blotting au moyen des anticorps monoclonaux Y6F5 et Y13F9 utilisés à la concentration de 1 µg/ml et d'un conjugué anti souris couplé à la phosphatase alcaline (Zymed ref :61-6422) diluée au 1/1000^{ème}. Les résultats ont montré qu'il existait une bande de 55 KD lorsque l'échantillon n'a pas été traité à la PNGase. Par contre, la bande a une masse plus faible lorsque l'échantillon a été préalablement traité à la PNGase

### 2.2 : caractérisation du motif glycosylé.

### 2.2.1 : Traitement de l'HA provenant de la souche de virus B/Jiangsu et extraction des glycanes

On a choisi l'anticorps monoclonal Y6F5 et la souche de virus B/Jiangsu purifiée par sédimentation sur gradient de saccharose pour cette caractérisation.

L'extraction de l'HA a été faite selon le même protocole que celui décrit dans le paragraphe précédent. Le virus résiduel a été éliminé par ultracentrifugation pendant 1 heure à 100000g à 4°C. L'HA se retrouvait dans le surnageant d'ultracentrifugation. L'HA a ensuite été dénaturée en présence de dodécyl sulfate de sodium (SDS) en ajoutant à une solution de 2,2 mg/ml de HA, 1mg/ml de dodécyl sulfate de sodium. Au bout de 5 minutes, on a ajouté du dithiotréitol (DTT) de façon à ce que la concentration finale en DTT dans le mélange soit 5mM. Après une incubation à 60°C pendant 15 minutes on a rajouté de l'iodoacétamide de façon à ce que la concentration finale en iodoacétamide dans le mélange soit de 10mM. Après une nouvelle incubation de 20 minutes dans l'obscurité à la température du laboratoire on a dialysé finalement le mélange contre un tampon PBS.

La glycoprotéine était alors sous une forme linéaire et les glycanes étaient mieux exposés pour un clivage enzymatique par la PNGase F.

L'HA dénaturée à une concentration d'environ 1 mg/ml a ensuite été traitée par la PNGase F (ref: Calbiochem ref: 362185) pendant 16 h à raison de 0.4 unités/mg dans un tampon phosphate 50mM, pH=7,5. Pendant la durée du traitement enzymatique on a contrôlé son activité enzymatique vis-à-vis de l'HA en réalisant une électrophorèse sur gel de polyacrylamide en présence de SDS. L'activité endoglycosidasique de la PNGase F entraine une diminution de massse de l'HA qui se caractérise sur gel de polyacrylamide par l'existence d'une bande de plus faible masse.

Après action de l'enzyme, on a précipité les protéines par un traitement à l'acide trichloracétique à 10%. Le surnageant qui contenait un mélange de glycanes a été finalement recueilli après centrifugation pendant 15min. à 10000g. En parallèle, on a effectué les mêmes traitements sur une aliquote de HA purifiée provenant du virus B/jiangsu purifié à l'exception du traitement à la PNGase. Cet échantillon a été utilisé comme contrôle négatif pour la suite des expériences.

### 2.2.2 : Purification des glycanes à l'aide du Biacore 3000

L'anticorps monoclonal Y6F5 à une concentration de 250µg/ml en tampon acétate 10mM, pH=4.5 a été fixé de façon covalente sur le sensorchip du Biacore 3000 en utilisant les instructions du fournisseur. L'échantillon de HA qui a été traité à la PNGase F ainsi que le contrôle négatif, non traité ont été ensuite injectés dans l'appareil.

Le signal des glycanes capturés par l'anticorps monoclonal observé est énorme (environ 1000 RU) bien que la quantité de matériel en jeu soit très faible (quelques µg). On n'a pas observé de signal avec l'échantillon qui sert de contrôle négatif.

On a ensuite désorbé du sensorchip les glycanes retenus par l'anticorps monoclonal Y6F5 en injectant quelques µl d'une solution de soude 5mM. On a récupéré 2µl d'une solution de glycanes à la sortie de l'appareil. L'expérience a été faite 2 fois pour avoir un volume suffisant.

On a ensuite ajouté à la solution de glycanes purifiés 1 ul d'acide Trifluoroacétique (TFA) avant d'analyser l'échantillon par spectrométrie de masse.

### 2.2.3 : caractérisation du motif glycosylé

L'analyse à été réalisée sur un appareil de type MALDI Tof (Autoflex II Bruker). Ce type d'appareil a été choisi en raison de la faible quantité d'analyte disponible. Les ions ont été détectés en mode réflectron négatif.

La préparation de la matrice, de l'analyte et son dépôt ont été réalisés comme suit : la matrice utilisée était l'acide dihydroxy benzoïque (DHB). La préparation de la matrice a été réalisée en préparant une solution saturante de matrice dans de l'acétone puis le surnageant a ensuite été dilué par l'ajout d'un volume d'eau. Un µl de cette solution de matrice a ensuite été mélangé à 1 µl d'analyte contenant les glycanes. 0,5µl de ce mélange a alors été déposé sur la plaque MALDI en acier inoxydable à deux endroits différents.

L'identification de la structure osidique a été réalisée par soustraction des spectres obtenus entre l'échantillon traité à la PNGase et l'échantillon non traité à la PNGase (contrôle négatif).

L'analyse comparative des deux spectres a permis de détecter un ion à la masse 1240 Da (cf figure 1). Nous avons ensuite calculé le nombre de sucres engagés dans cette structure sur la base des structures des glycanes de la grippe décrites notamment par Keil W et coll. (Virology, 133, 77-91 (1984) et plus récemment par Mir-Shekari S.Y. et coll. (Journal of biological Chemistry, 272, 4027-4036 (1997). On s'est appuyé sur la structure de la séquence oligosaccharidique de base : (glucose)₃-(mannose)₉-(N-acteyl-glucosamine)₂ sous sa forme triantennée qui est transférée par l'intermédiaire du dolichol phosphate et à partir de laquelle s'opère les processus de transformation qui se traduisent généralement par la perte des résidus glucose, par la perte de résidus mannose à des degrés variables et par l'introduction de nouveaux résidus sucrés qui sont le N-acetyl glucosamine, le galactose et le fucose qui se greffent sur les structures de base modifiées pour déterminer la structure de l'oligosaccharide reconnu par l'anticorps monoclonal Y6F5 sur la base de son poids moléculaire de 1240 daltons. En étudiant toutes les combinaisons de résidus sucrés possibles, on est arrivé à la conclusion qu'il y en avait aucune qui permettait de retomber sur la masse de notre ion. On a alors considéré qu'un résidu non sucré puisse être également présent sur le motif glycosidique reconnu par l'anticorps monoclonal Y6F5. notamment un groupement sulfate.

Pour étayer cette hypothèse, la solution de glycanes purifiés récupérée à la sortie de l'appareil a été traitée avec un volume d'une solution d'ammoniaque 1M à 60°C pendant 1 heure. Ce traitement avait pour but d'éliminer les groupements sulfates éventuellement fixés sur des résidus sucrés. L'échantillon obtenu a ensuite été traité et analysé de la même manière.

Comme le montre la figure 1 (spectre du haut), nous remarquons une perte de masse de 80 Da caractéristique de la perte d'un groupement sulfate. Cela confirme donc l'existence d'un groupement sulfate sur le motif glycosylé reconnu par l'anticorps monoclonal Y6F5. Le spectre de l'échantillon de glycanes purifiés non traités à l'ammoniaque montre deux pics d'intérêt (à 1240 et à environ 1160) (cf. spectre du bas sur la figure 1) ; cela est du à la labilité du groupement sulfate lors de l'ionisation de l'analyte.

On observe également que les deux spectres ne sont pas de qualité équivalente. Cela s'explique par le fait que, outre la dilution occasionnée lors de l'expérience de désulfatation, celle ci a amené des groupements ammoniums qui se sont substitués aux sels déjà présents. La combinaison de ces deux phénomènes fait que l'on observe majoritairement l'ion moléculaire et beaucoup moins les adduits.

Sur la base du PM du motif glycanique isolé par immunoaffinité à l'aide de l'anticorps monoclonal Y6F5 et des expériences de désulfatation, on en a déduit que le motif glycanique contient 7 sucres. Dans ce cas le PM de l'ensemble de ces 7 sucres, qui est de 1160 Da correspond au PM du motif glycanique désulfaté déterminé par spectrométrie de masse. Le groupement sulfate est relié au galactose car les cellules dans lesquelles se reproduit le virus grippal renferment une enzyme de sulfatation du galactose (galactose sulfatase).

## Revendications

1. Un anticorps monoclonal spécifique de l'hémagglutinine du virus grippal, reconnaissant un oligosaccharide contenant un galactose sulfate présent à la fois sur la partie N-glycosylée des sous types H1 et H3 des hémagglutinines des virus grippaux de type A et de l'hémagglutinine des virus grippaux de type B, les dits virus ayant été produits sur du matériel biologique d'origine aviaire.

2. Un anticorps monoclonal selon la revendication 1, selon laquelle la structure antigénique est présente sur l'hémagglutinine des virus grippaux de type B appartenant au groupe B/victoria.

3. Un anticorps monoclonal selon la revendication 1 ou 2, selon laquelle la structure antigénique est présente sur l'hémagglutinine des virus grippaux de type B appartenant au groupe B/yagamata.

4. Un anticorps monoclonal selon la revendication 1, selon laquelle le poids moléculaire de l'oligosaccharide est d'environ 1240 daltons.

5. Un anticorps monoclonal selon l'une des revendications 1 à 4, **caractérisé en ce qu'**il est produit par l'hybridome Y6F5 déposé à la Collection Nationale de Cultures de Microorganismes sous le numéro d'enregistrement CNCM I-3787.

6. Un anticorps monoclonal selon l'une des revendications 1 à 5, sous une forme humanisée.

7. Un fragment ou dérivé d'anticorps monoclonal selon l'une des revendications 1 à 6, choisi dans le groupe des fragments d'anticorps constitué de Fab, Fab', F(ab)'₂ et scFv.

8. Une méthode de détection du virus grippal ou de l'hémagglutinine du virus grippal dans un matériel biologique comprenant une étape selon laquelle on met le dit matériel en contact avec un anticorps monoclonal selon l'une des revendications 1 à 6, ou un fragment ou un dérivé de celui ci selon la revendication 7, de façon à former des complexes antigène/anticorps suite à l'interaction spécifique entre ledit anticorps monoclonal ou fragment ou dérivé de celui ci et l'hémagglutinine du virus de la grippe contenue dans ledit matériel biologique, et une étape où on met en évidence la présence de ces complexes antigène/anticorps.

9. Une méthode selon la revendication 8, comprenant une étape préalable selon laquelle on met le matériel en contact avec un support solide sur lequel est fixé un agent de capture reconnaissant spécifiquement une structure du virus grippal.

10. Une méthode de dosage de l'hémagglutinine du virus grippal contenue dans un matériel biologique qui comprend les étapes suivantes :
a) on met le matériel en contact avec un support solide sur lequel est fixé un premier anticorps monoclonal spécifique reconnaissant une structure protéique de l'hémagglutinine du virus grippal ;
b) on retient spécifiquement sur le support solide les complexes antigène/anticorps qui résultent de l'interaction spécifique de cet anticorps monoclonal avec l'hémagglutinine du virus grippal contenue dans le matériel biologique.
c) on met en contact les complexes antigène/anticorps retenus sur le support solide avec un deuxième anticorps monoclonal selon l'une des revendications 1 à 6 ou un fragment ou un dérivé de celui-ci selon la revendication 7 et portant directement ou indirectement un marqueur ;
d) on mesure la quantité de marqueur qui s'est fixé spécifiquement sur les complexes antigène/anticorps ; et
e) à partir de la quantité de marqueur mesurée, on détermine la quantité d'hémagglutinine contenue dans le matériel par comparaison avec un ou plusieurs réactifs de référence.

11. Une méthode de dosage selon la revendication 10, selon laquelle le matériel est une préparation vaccinale contenant de l'hémagglutinine provenant d'une ou plusieurs souches de virus grippaux.

12. Une méthode de dosage de l'hémagglutinine du virus grippal contenue dans un matériel biologique qui comprend les étapes suivantes :
a) on met le matériel en contact avec un support solide sur lequel est fixé un premier anticorps monoclonal selon l'une des revendications 1 à 6 ou un fragment ou un dérivé de celui-ci selon la revendication 7 ;
b) on retient spécifiquement sur le support solide les complexes antigène-anticorps qui résultent de l'interaction spécifique de l'anticorps monoclonal avec l'hémagglutinine du virus grippal contenue dans le matériel;
c) on met en contact les complexes antigène/anticorps retenus sur le support solide avec un deuxième anticorps monoclonal selon l'une des revendications 1 à 6 ou un fragment ou un dérivé de celui-ci selon la revendication 7 portant directement ou indirectement un marqueur, le deuxième anticorps monoclonal pouvant être distinct ou identique au premier anticorps;
d) on mesure la quantité de marqueur qui s'est fixé sur les complexes antigène/anticorps ; et
e) à partir de la quantité de marqueur mesurée, on détermine la quantité d'hémagglutinine contenue dans le matériel par comparaison avec un ou plusieurs réactifs de référence.

13. Une méthode de dosage selon la revendication 12, selon laquelle le matériel biologique est une préparation vaccinale purifiée contenant de l'hémagglutinine provenant d'une ou plusieurs souches de virus grippaux.

14. Une méthode de dosage selon l'une des revendications 8 à 13, selon laquelle le matériel biologique est d'origine aviaire.

15. Un procédé de purification du virus de la grippe ou de l'hémagglutinine du virus grippal à partir d'un matériel biologique d'origine aviaire qui comprend les étapes suivantes:
a. on met le matériel en contact avec un support solide sur lequel est fixé un premier anticorps monoclonal selon l'une des revendications 1 à 6 ou un fragment ou un dérivé de celui-ci selon la revendication 7 ;
b. on retient spécifiquement sur le support solide les complexes antigène/anticorps qui résultent de l'interaction spécifique de l'anticorps monoclonal avec l'hémagglutinine du virus grippal contenue dans le matériel;
c. on libère le virus de la grippe ou l'hémagglutinine du virus grippal des complexes antigène-anticorps retenus sur le support solide ; et
d. on récupère le virus de la grippe ou l'hémagglutinine du virus grippal.

16. Une composition pharmaceutique comprenant un anticorps monoclonal selon l'une des revendications 1 à 6 ou un dérivé ou un fragment de celui-ci selon la revendication 7.

17. Utilisation d'un anticorps monoclonal selon l'une des revendications 1 à 6, ou d'un fragment ou d'un dérivé de celui-ci selon la revendication 7, pour la purification d'un l'oligosaccharide sulfaté selon l'une des revendications 1 à 4.

## Patentansprüche

1. Monoklonaler Antikörper, der für das Hämagglutinin des Influenzavirus spezifisch ist und ein ein Galactosesulfat enthaltendes Oligosaccharid erkennt, das zugleich auf dem N-glykosylierten Anteil der Subtypen H1 und H3 der Hämagglutinine der Influenzaviren Typ A und des Hämagglutinins der Influenzaviren Typ B vorliegt, wobei die Viren auf aus Vögeln stammendem biologischem Material hergestellt werden.

2. Monoklonaler Antikörper nach Anspruch 1, wobei die antigene Struktur auf dem Hämagglutinin der Influenzaviren Typ B, die zur Linie B/Victoria gehören, vorliegt.

3. Monoklonaler Antikörper nach Anspruch 1 oder 2, wobei die antigene Struktur auf dem Hämagglutinin der Influenzaviren Typ B, die zur Linie B/Yamagata gehören, vorliegt.

4. Monoklonaler Antikörper nach Anspruch 1, wobei das Molekulargewicht des Oligosaccharids etwa 1240 Dalton beträgt.

5. Monoklonaler Antikörper nach einem der Ansprüche 1 bis 4, **dadurch gekennzeichnet, dass** er durch das Hybridom Y6F5 produziert wird, das bei der Collection Nationale de Cultures de Microorganismes unter der Registrierungsnummer CNCM 1-3787 hinterlegt ist.

6. Monoklonaler Antikörper nach einem der Ansprüche 1 bis 5 in einer humanisierten Form.

7. Fragment oder Derivat eines monoklonalen Antikörpers nach einem der Ansprüche 1 bis 6, ausgewählt aus der Gruppe der Antikörperfragmente, die aus Fab, Fab', F(ab)'₂ und scFv besteht.

8. Verfahren zum Nachweis des Influenzavirus oder des Hämagglutinins des Influenzavirus in einem biologischen Material, umfassend einen Schritt, bei dem man das Material mit einem monoklonalen Antikörper nach einem der Ansprüche 1 bis 6 oder einem Fragment oder Derivat davon nach Anspruch 7 in Kontakt bringt, so dass Antigen/Antikörper-Komplexe nach der spezifischen Wechselwirkung zwischen dem monoklonalen Antikörper oder dem Fragment oder Derivat davon und dem in dem biologischen Material enthaltenen Hämagglutinin des Influenzavirus gebildet werden, und einen Schritt, bei dem man das Vorliegen dieser Antigen/Antikörper-Komplexe nachweist.

9. Verfahren nach Anspruch 8, das einen vorausgehenden Schritt umfasst, bei dem man das Material mit einem festen Träger in Kontakt bringt, auf dem ein Einfangmittel gebunden ist, das spezifisch eine Struktur des Influenzavirus erkennt.

10. Verfahren zur Messung des in einem biologischen Material enthaltenen Hämagglutinins des Influenzavirus, das folgende Schritte umfasst:
a) man bringt das Material in Kontakt mit einem festen Träger, auf dem ein erster monoklonaler Antikörper gebunden ist, der spezifisch eine Proteinstruktur des Hämagglutinins des Influenzavirus erkennt,
b) man hält auf dem festen Träger spezifisch die Antigen/Antikörper-Komplexe zurück, die durch die spezifische Wechselwirkung dieses monoklonalen Antikörpers mit dem in dem biologischen Material enthaltenen Hämagglutinin des Influenzavirus gebildet werden,
c) man bringt die auf dem festen Träger zurückgehaltenen Antigen/Antikörper-Komplexe mit einem zweiten monoklonalen Antikörper nach einem der Ansprüche 1 bis 6 oder einem Fragment oder Derivat davon nach Anspruch 7 in Kontakt, der/das direkt oder indirekt einen Marker trägt,
d) man misst die Menge an Marker, die spezifisch an die Antigen/Antikörper-Komplexe bindet, und
e) man bestimmt ausgehend von der gemessenen Menge an Marker die Menge an Hämagglutinin, die in dem Material enthalten ist, durch Vergleich mit einem oder mehreren Referenzreagenzien.

11. Messverfahren nach Anspruch 10, wobei das Material eine Impfstoffzubereitung ist, die aus einer oder mehreren Quellen für Influenzavirus stammendes Hämagglutinin enthält.

12. Verfahren zur Messung des in einem biologischen Material enthaltenen Hämagglutinins des Influenzavirus, das folgende Schritte umfasst:
a) man bringt das Material in Kontakt mit einem festen Träger, auf dem ein erster monoklonaler Antikörper nach einem der Ansprüche 1 bis 6 oder ein Fragment oder Derivat davon nach Anspruch 7 gebunden ist,
b) man hält auf dem festen Träger spezifisch die Antigen/Antikörper-Komplexe zurück, die durch die spezifische Wechselwirkung des monoklonalen Antikörpers mit dem in dem Material enthaltenen Hämagglutinin des Influenzavirus gebildet werden,
c) man bringt die auf dem festen Träger zurückgehaltenen Antigen/Antikörper-Komplexe mit einem zweiten monoklonalen Antikörper nach einem der Ansprüche 1 bis 6 oder einem Fragment oder Derivat davon nach Anspruch 7 in Kontakt, der/das direkt oder indirekt einen Marker trägt, wobei der zweite monoklonale Antikörper von dem ersten Antikörper verschieden oder mit diesem identisch sein kann,
d) man misst die Menge an Marker, die spezifisch an die Antigen/Antikörper-Komplexe bindet, und
e) man bestimmt ausgehend von der gemessenen Menge an Marker die Menge an Hämagglutinin, die in dem Material enthalten ist, durch Vergleich mit einem oder mehreren Referenzreagenzien.

13. Messverfahren nach Anspruch 12, bei dem das biologische Material eine gereinigte Impfstoffzubereitung ist, die das aus einem oder mehreren Influenzavirus-Stämmen stammende Hämagglutinin enthält.

14. Messverfahren nach einem der Ansprüche 8 bis 13, wobei das biologische Material aus Vögeln stammt.

15. Verfahren zur Reinigung des Influenzavirus oder des Hämagglutinins des Influenzavirus aus einem aus Vögeln stammenden biologischen Material, umfassend die folgenden Schritte:
a) man bringt das Material in Kontakt mit einem festen Träger, auf dem ein erster monoklonaler Antikörper nach einem der Ansprüche 1 bis 6 oder ein Fragment oder Derivat davon nach Anspruch 7 gebunden ist,
b) man hält auf dem festen Träger spezifisch die Antigen/Antikörper-Komplexe zurück, die durch die spezifische Wechselwirkung des monoklonalen Antikörpers mit dem in dem Material enthaltenen Hämagglutinin des Influenzavirus gebildet werden,
c) man setzt das Influenzavirus oder das Hämagglutinin des Influenzavirus aus den auf dem festen Träger zurückgehaltenen Antigen/Antikörper-Komplexen frei und
d) man erhält das Influenzavirus oder das Hämagglutinin des Influenzavirus.

16. Pharmazeutische Zusammensetzung, die einen monoklonalen Antikörper nach einem der Ansprüche 1 bis 6 oder ein Derivat oder Fragment davon nach Anspruch 7 umfasst.

17. Verwendung eines monoklonalen Antikörpers nach einem der Ansprüche 1 bis 6 oder eines Fragments oder Derivats davon nach Anspruch 7 zur Reinigung eines sulfatierten Oligosaccharids nach einem der Ansprüche 1 bis 4.

## Claims

1. Flu virus hemagglutinin-specific monoclonal antibody which recognizes an oligosaccharide containing a galactose sulphate present on the N-glycosylated part of the H1 and H3 subtypes of the hemagglutinins of type A flu viruses and of the hemagglutinin of type B flu viruses, said viruses having been produced on biological material of avian origin.

2. Monoclonal antibody according to Claim 1, according to which the antigenic structure is present on the hemagglutinin of type B flu viruses belonging to the B/Victoria group.

3. Monoclonal antibody according to Claim 1 or 2, according to which the antigenic structure is present on the hemagglutinin of type B flu viruses belonging to the B/Yagamata group.

4. Monoclonal antibody according to Claim 1, according to which the molecular weight of the oligosaccharide is approximately 1240 daltons.

5. Monoclonal antibody according to one of Claims 1 to 4, which is produced by the Y6F5 hybridoma deposited with the Collection Nationale de Cultures de Microorganismes [National Collection of Microorganism Cultures] under the registration number CNCM 1-3787.

6. Monoclonal antibody according to one of Claims 1 to 5, in a humanized form.

7. Fragment or derivative of the monoclonal antibody according to one of Claims 1 to 6, selected from the group of antibody fragments consisting of Fab, Fab', F(ab)'₂ and scFv.

8. Method for detecting flu virus or flu virus hemagglutinin in a biological material, comprising a step according to which said material is brought into contact with a monoclonal antibody according to one of Claims 1 to 6, or a fragment or a derivative thereof according to Claim 7, so as to form antigen/antibody complexes subsequent to the specific interaction between said monoclonal antibody, or fragment or derivative thereof, and the flu virus hemagglutinin contained in said biological material, and a step in which the presence of these antigen/antibody complexes is revealed.

9. Method according to Claim 8, comprising a prior step according to which the material is brought into contact with a solid support to which is bound a capture agent which specifically recognizes a flu virus structure.

10. Method for assaying flu virus hemagglutinin contained in a biological material, which comprises the following steps:
a) the material is brought into contact with a solid support to which is bound a first specific monoclonal antibody which recognizes a protein structure of the flu virus hemagglutinin;
b) the antigen/antibody complexes which result from the specific interaction of this monoclonal antibody with the flu virus hemagglutinin contained in the biological material are specifically retained on the solid support;
c) the antigen/antibody complexes retained on the solid support are brought into contact with a second monoclonal antibody according to one of Claims 1 to 6, or a fragment or a derivative thereof according to Claim 7, directly or indirectly carrying a label;
d) the amount of label which has bound specifically to the antigen/antibody complexes is measured; and
e) based on the amount of label measured, the amount of hemagglutinin contained in the material is determined by comparison with one or more reference reagents.

11. Assaying method according to Claim 10, according to which the material is a vaccinal preparation containing hemagglutinin originating from one or more flu virus strains.

12. Method for assaying flu virus hemagglutinin contained in a biological material, which comprises the following steps:
a) the material is brought into contact with a solid support to which is bound a first monoclonal antibody according to one of Claims 1 to 6, or a fragment or a derivative thereof according to Claim 7;
b) the antigen-antibody complexes which result from the specific interaction of the monoclonal antibody with the flu virus hemagglutinin contained in the material are specifically retained on the solid support;
c) the antigen/antibody complexes retained on the solid support are brought into contact with a second monoclonal antibody according to one of Claims 1 to 6, or a fragment or a derivative thereof according to Claim 7, directly or indirectly carrying a label, it being possible for the second monoclonal antibody to be different than or identical to the first antibody;
d) the amount of label which has bound to the antigen/antibody complexes is measured; and
e) based on the amount of label measured, the amount of hemagglutinin contained in the material is determined by comparison with one or more reference reagents.

13. Assaying method according to Claim 12, according to which the biological material is a purified vaccinal preparation containing hemagglutinin originating from one or more flu virus strains.

14. Assaying method according to one of Claims 8 to 13, according to which the biological material is of avian origin.

15. Method for purifying flu virus or flu virus hemagglutinin from a biological material of avian origin, which comprises the following steps:
a. the material is brought into contact with a solid support to which is bound a first monoclonal antibody according to one of Claims 1 to 6, or a fragment or a derivative thereof according to Claim 7;
b. the antigen/antibody complexes which result from the specific interaction of the monoclonal antibody with the flu virus hemagglutinin contained in the material are specifically retained on the solid support;
c. the flu virus or the flu virus hemagglutinin is released from the antigen-antibody complexes retained on the solid support; and
d. the flu virus or the flu virus hemagglutinin is recovered.

16. Pharmaceutical composition comprising a monoclonal antibody according to one of Claims 1 to 6, or a derivative or a fragment thereof according to Claim 7.

17. Use of a monoclonal antibody according to one of Claims 1 to 6, or of a fragment or a derivative thereof according to Claim 7, for the purification of a sulphated oligosaccharide according to one of Claims 1 to 4.
